# EUROPEAN PATENT SPECIFICATION

(11) **EP 1 512 428 B1**
(45) Date of publication and mention of the grant of the patent: **26.03.2008**
(21) Application number: 04255369.3
(22) Date of filing: 03.09.2004
(51) Int. Cl.: A61M 27/00, A61B 5/03

(54) **Apparatus for controlling normal pressure hydrocephalus**
Vorrichtung zum Regeln eines Normaldruck-Hydrocephalus
Dispositif pour régler une hydrocéphalie de pression normale

(30) Priority: 05.09.2003 US 656973
(43) Date of publication of application: 09.03.2005
(73) Proprietor: Codman & Shurtleff, Inc., Raynham, Massachusetts 02767-0350 (US)
(72) Inventor: Rosenberg, Meir, Newton, MA 02459 (US)
(74) Representative: Mercer, Christopher Paul

(56) References cited:
- WO-A-98/35610
- DE-A- 10 033 138
- DE-C- 19 643 782

## Description

### FIELD OF THE INVENTION

The present invention relates to apparatus for managing hydrocephalus in a patient. More particularly, the invention relates to apparatus for draining cerebrospinal fluid in a hydrocephalus patient at a rate responsive to volumetric variations in the patient's ventricular cavity. Even more particularly, the invention relates to a shunt system having an adjustable resistance valve and a volume sensor for regulating the drainage of cerebrospinal fluid in a ventricular cavity undergoing volumetric variations. There is also disclosed a method of using such a shunt system to manage cerebrospinal fluid flow in patients afflicted with normal pressure hydrocephalus.

### BACKGROUND OF THE INVENTION

Hydrocephalus is a condition afflicting patients who are unable to regulate cerebrospinal fluid flow through their body's own natural pathways. Cerebrospinal fluid (CSF) is normally produced by the choroid plexus of the brain and carries essential nutrients, hormones, and other cellular components to various portions of the brain as the fluid circulates through the ventricular system. Along the way, the CSF also helps absorb shock and cushions the brain as the fluid diffuses over the brain and spinal cord. Cerebrospinal fluid that is not recirculated eventually drains into the sagittal sinus where it is naturally absorbed by the body's venous system. In a patient suffering from hydrocephalus, the CSF absorption rate fails to keep up with the production rate, either because of an obstruction along the natural CSF pathway or due to diseased choroid plexus which increases CSF formation. The unabsorbed or excess CSF accumulates in the ventricles of the patient's brain, leading to an increase in intracranial pressure. If left untreated, the increased intracranial pressure can lead to serious medical conditions such as compression of the brain tissue and impaired blood flow to the brain, with such potential consequences as coma and/or death.

The conventional treatment for hydrocephalus patients has involved draining the excess fluid away from the ventricles and rerouting the excess cerebrospinal fluid to another area of the patient's body, such as the peritoneum or vascular system- An implantable drainage system, commonly referred to as a shunt, is often used to carry out the transfer of fluid and restore the balance between the formation and absorption of CSF in the patient. In order to install the shunt system, a scalp incision is made and a small hole is drilled in the skull. A proximal, or ventricular catheter is installed in the ventricular cavity of the patient's brain, while a distal, or drainage catheter is installed in that portion of the patient's body where the excess fluid is to be reintroduced.

To regulate the flow of cerebrospinal fluid between the proximal and distal ends of the shunt system, the main body of the shunt usually includes a pump or one-way control valve. Generally, shunt systems include a valve mechanism that operates by permitting fluid flow only once the fluid pressure reaches a certain threshold level. That is, fluid enters the valve only when the fluid pressure overcomes the valve mechanism's resistance to open. Some valve mechanisms permit the non-invasive adjustment, or programming, of the opening pressure level at which fluid flow commences. These passive relief valves can generally be characterized as falling within one of two categories. Differential pressure valves regulate the differential pressure across the shunt, i.e., the difference of the pressures captured at the proximal and distal ends of the shunt system. Variable resistance valves regulate the flow of CSF through the shunt by varying the resistance of the valve, i.e., adjusting the ratio between differential pressure across the valve and the CSF flow through the valve.

Shunts having valve mechanisms that continuously drain CSF are well known, as are shunts with valves that control and/or adjust the opening pressure and/or drainage rate of the patient's CSF. In congenital hydrocephalus patients (i.e., the hydrocephalus is acquired at birth), and especially in pediatric patients, such current shunt devices have proven to be successful in regulating CSF flow. However, these same shunt devices have been less effective in patients who suffer from idiopathic normal pressure hydrocephalus, a type of hydrocephalus usually acquired in the later stages of life. Normal pressure hydrocephalus is characterized by enlarged ventricles in the patient's brain, though the ventricles themselves may register as having "normal" pressure. Normal pressure hydrocephalus typically affects middle-aged to elderly adults, as the disease itself is often due to the onset of old age. Classic symptoms include dementia, gait instability, and urinary incontinence. Because the disease produces a "normal" pressure reading, diagnosis is made by correlating the patient symptoms with the size of the ventricles via CT and/or MRI, rather than by detecting the pressure within the ventricular cavities. Presumably in normal pressure hydrocephalus, the pressure in the ventricles was at some time sufficient to cause them to dilate. Once the ventricles expanded, the pressure of the fluid within them returned to "normal" pressure. In older patients, ventricular compliance becomes reduced and so the ventricles are no longer able to contract back to their original size or volume in an appropriate amount of time. As a result, the ventricles remain enlarged, and the patient suffers from neurological dysfunction due to the compression of the neighboring parts of the brain by the overexpanded ventricles.

Most of the currently available shunt devices are configured to be responsive to changes in proximal, distal and/or atmospheric pressure, i.e., the shunts respond to variations in proximal and distal pressures and/or atmospheric pressure. Whereas these shunts have been effective in regulating cerebrospinal fluid in congenital hydrocephalus patients, these shunts have not proven as effective in patients having normal pressure hydrocephalus because the adjusted opening pressure levels do not account for changes in the volume of the ventricular cavity being drained. Since normal pressure hydrocephalus patients experience more pronounced volumetric variations in their ventricular cavities than ventricular pressure variations, the passive relief valves currently available are less effective in these patients who require drainage rate adjustment based on perceived changes in volume. There is thus a need for a shunt system that can adjust its drainage rate or valve resistance in response to changes in ventricular volume as well as pressure changes in the ventricular cavity, in order to achieve a desirable drainage rate, and a method for managing CSF flow in normal pressure hydrocephalus patients using such a shunt system.

DE 100 33 138 and DE 196 43782 each disclose apparatus of the type set out in the preamble of the accompanying claim 1.

### SUMMARY OF THE INVENTION

The present invention achieves the aforementioned goals by providing an adjustable drainage system for regulating CSF flow in a hydrocephalus patient where the drainage rate is adjusted in response to variations in the ventricular volume of the patient. The system includes an adjustable resistance valve and a volume sensor that can be periodically energized with an external system controller device by the patient or attending physician to determine when, or if, a change in the ventricular volume has occurred. The system enables the user to adjust the valve's resistance in response to changes in the ventricular volume using the controller device so that a target ventricular volume can be achieved. Also disclosed although outside the scope of the invention, is a method of regulating the drainage of CSF from the cranial cavity of the patient using the system of the present invention.

The present invention provides an apparatus for regulating CSF flow in a hydrocephalus patient which comprises an implantable shunt system having an adjustable resistance valve for regulating the flow of CSF into and out of a ventricular cavity of the patient, and includes a sensor element for measuring a physiological characteristic of the patient. The sensor element can be coupled to the valve, or it can be separate from the valve itself. The apparatus also comprises an external system controller device that is selectively operable for energizing and communicating with the implantable shunt system. In operation, the system controller device is configured to effect an adjustment of the resistance of the valve when the device is applied to the patient (i.e., when the system is energized by the system controller device). The sensor element is a volume sensor for detecting volumetric variations within the ventricular cavity of the patient.

The implantable shunt system and external system controller device of the present apparatus are configured to communicate data between one another during operation (i.e., when the device is applied to the patient and the implanted shunt system is energized). For instance, the system controller device can be configured to energize and receive an input signal generated from the sensor element that is representative of the measured value of the physiological characteristic. In one aspect, the physiological characteristic is a measured volume of the ventricular cavity of the patient. The system controller device can also be configured to generate and transmit to the valve an output control signal that commands the valve to adjust its resistance.

In another aspect of the invention, the system controller device can include a processing unit such as a microprocessor which enables the device to compare the measured volume detected by the volume sensor to a predetermined target volume for the patient. The predetermined target value can be ascertained through clinical assessment of the patient and is therefore customized for each particular patient. This target value is then preset or programmed into the system controller device. If, during operation, the system controller device detects a difference between the measured value and the target value, the microprocessor is programmed to increase or decrease the resistance for the valve, depending on whether the measured value is higher or lower than the target value, in order to maintain the target ventricular volume for the patient over time. To adjust the valve, the microprocessor can generate an output control signal to the valve which commands it to adjust its current resistance to the desired resistance. If, however, the measured value is the same as, or falls within an acceptable range of the target value, then the system controller device is programmed to make no changes to the resistance level. To safeguard against repeated or excessive valve adjustments within a short window of time, which could produce deleterious health consequences for the patient, the system controller device can include a timed shutoff mechanism which would limit the user's ability to adjust the valve with the system controller device. For example, the system controller device's valve adjustment features can be configured to deactivate after each use until a preset amount of time (e.g., a day, two days, a week, etc.) has passed whereby the valve adjustment feature can be automatically reactivated.

In yet another aspect of the invention, the implantable shunt system can further include a second sensor element for measuring an additional physiological characteristic of the patient. Like the first sensor element, the second sensor element can be configured to transmit data representing the measured value of the second physiological characteristic to the system controller device. The second sensor element can be coupled to the valve, or it can be separate from the valve itself. In an exemplary embodiment, the second sensor clement is a pressure sensor and the additional physiological characteristic is ventricular pressure of the patient.

Further features of the invention, its nature and various advantages, will be more apparent from the accompanying drawings and the following detailed description of the drawings and the preferred embodiments.

### BRIEF DESCRIPTION OF THE DRAWINGS

The invention can be more fully understood from the following detailed description taken in conjunction with the accompanying drawings, in which:
FIG- I is a perspective view of the shunt system and system controller device of the present invention;

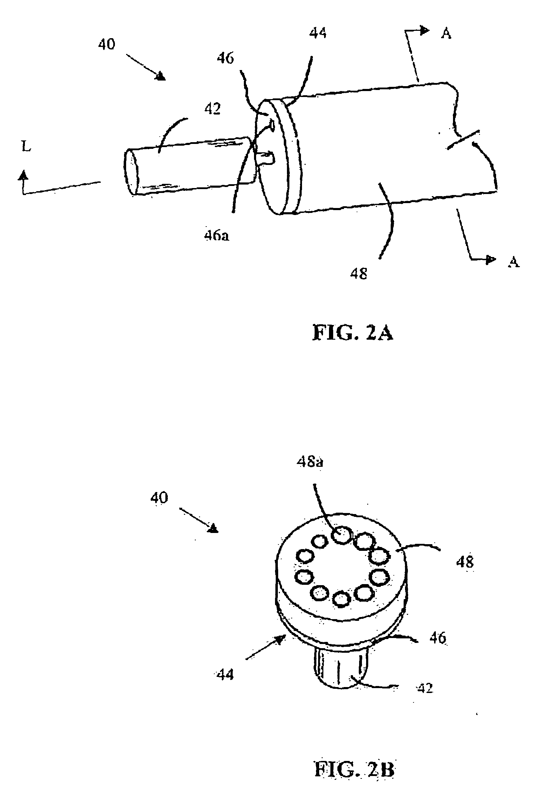

FIG. 2A is a side perspective view of an embodiment of the adjustable resistance valve of the present invention; and
FIG. 2B is a cross-sectional perspective view of the adjustable resistance valve of FIG. 2A along lines A-A.

### DETAILED DESCRIPTION OF THE INVENTION

Described herein is a method and apparatus for regulating CSF flow in a hydrocephalus patient by adjusting the drainage rate of excess CSF in response to variations in the ventricular volume of the patient. The method, which is outside the scope of the present invention, and apparatus of the present invention regulates the drainage of CSF from the cranial cavity of a hydrocephalus patient. The apparatus includes an adjustable valve and a volume sensor that can be periodically energized by the patient or attending physician to determine when, or if, a change in the ventricular volume has occurred, and whether corrective adjustment of the valve's resistance should be made.

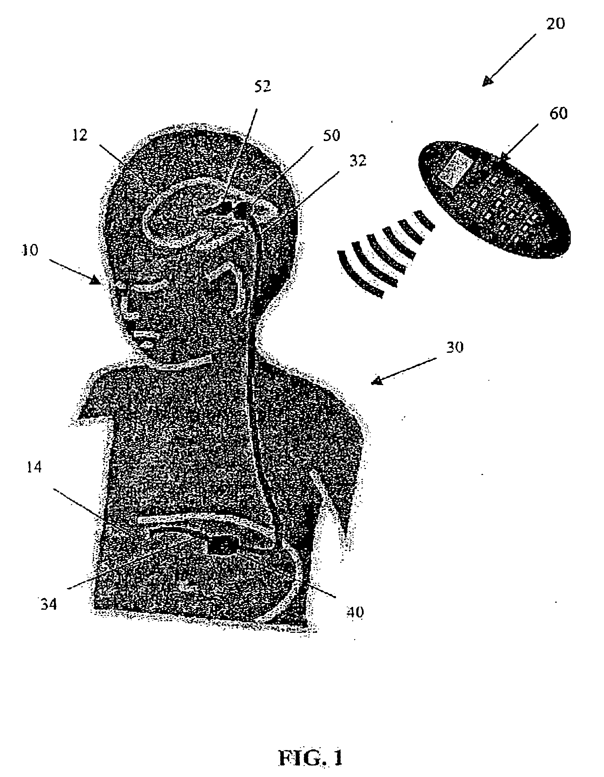

Turning now to the drawings and particularly to FIG. 1, an exemplary embodiment of an apparatus 20 in accordance with the present invention is illustrated. The apparatus 20 comprises a shunt system 30 shown herein as being implanted within a hydrocephalus patient 10. The implantable shunt system 30 includes a proximal or ventricular catheter 32 installed in a ventricular cavity 12 of the patient 10, and a distal or drainage catheter 34 installed in the peritoneum 14 of the patient 10. Extending between the ventricular and drainage catheters 32, 34 is an adjustable resistance valve 40 for regulating the flow of CSF into and out of the ventricular cavity 12 of the patient 10. The valve 40 can be located anywhere along the fluid pathway of the shunt system 30 such as in the patient's cranium. Preferably, the valve 40 is located within the peritoneal cavity 14 of the patient 10 as illustrated, so that size constraints for the valve 40 are minimized (i.e., larger valves 40 can be implanted within the peritoneum than adjacent to the skull).

Also included with the apparatus 20 is a sensor element 50 for measuring a physiological characteristic of the patient. The sensor element 50 can be coupled to the valve 40, or it can be separate from the valve 40 as shown in FIG. 1. Further, while the sensor element 50 is shown as being positioned within the CSF flow pathway of the shunt system 30, it is understood that sensor element 50 can be located outside of the CSF flow pathway though still residing within the ventricular cavity 12 of the patient 10. The sensor element 50 is a volume sensor for detecting volumetric variations within the ventricular cavity 12 of the patient 10. The apparatus 20 also comprises an external system controller device 60 that is selectively operable for energizing and communicating with the implantable shunt system 30. In operation, the system controller device 60 is configured to effect an adjustment of the resistance of the valve 40 when the device 60 is applied to the patient (i.e., when the system 30 is energized by the system controller device 60).

In the present invention, the implantable shunt system 30 and external system controller device 60 are configured to communicate data between one another when in operation (i.e., when the device 60 is applied to the patient and the implanted shunt system 30 is energized). For example, the system controller device 60 can be configured to energize and receive an input signal generated from the sensor element 50 that is representative of the measured value of the physiological characteristic. The sensor element 50 is a volume sensor and hence the physiological characteristic is a measured volume of the ventricular cavity 12 of the patient 10. The system controller device 60 can also be configured to generate and transmit to the valve 40 an output control signal that commands the valve 40 to adjust its resistance.

The implantable shunt system 30 of the present invention can further include a second sensor clement 52 for measuring an additional physiological characteristic of the patient. Like the first sensor element 50, the second sensor element 52 can be configured to transmit data representing the measured value of the second physiological characteristic to the system controller device 60. The second sensor element 52 can be coupled to the valve 40, or it can be separate from the valve 40 itself. In an exemplary embodiment, the second sensor element 52 is a pressure sensor and the additional physiological characteristic is ventricular pressure of the patient 10.

The shunt system 30 and system controller device 60 of the present invention can be equipped with electronic circuitry similar to those for medical telemetry systems that communicate physiological data (e.g., temperature, pressure, etc.) between an implant and a receiver unit. For example, the sensor element 50 can be configured to generate an analog data signal that is then converted electronically to a digital pulse which is then transmitted by radiofrequency (RF) to the system controller device 60. One skilled in the art will recognize that these are merely examples of the forms of remote communication suitable for the present invention, and that other forms of non-invasive communication can be utilized without departing from the scope of the present invention.

In another aspect of the invention, the system controller device 60 can include a processing unit (e.g., a microprocessor) which enables the device 60 to compare the measured volume detected by the sensor element 50 to a predetermined target volume for the patient 10. The predetermined target value can be ascertained through clinical assessment of the patient 10 and is therefore customized for each particular patient. This target value is then preset or programmed into the system controller device 60. During operation, the system controller device 60 energizes the shunt system 30 and detects the measured value of the physiological characteristic. The device 60 operates according to an algorithm which ascertains whether the measured value is higher than, lower than, or within an acceptable range of the target value. Based on this assessment, the algorithm will then determine whether the resistance should be increased, decreased or maintained accordingly in order to achieve the target ventricular volume for the patient 10. For instance, the valve's resistance is decreased if the measured volume is higher than the target volume; conversely, the resistance of the valve 40 is increased if the measured volume is lower than the target volume. The microprocessor can then generate an output control signal to the valve 40 which commands it to adjust its current resistance to the desired resistance. If the measured value is essentially the same as, or within an acceptable range of the target value, then the current resistance is maintained and no changes are made.

In accordance with one embodiment of the present invention, the adjustable resistance valve 40 includes an actuator 42 as shown in FIGS. 2A and 2B. The actuator 42 allows the selection of the resistance to flow of the valve 40. The actuator 42 is coupled to a selection mechanism 44 comprising a disc 46 having at least one aperture 46a traversing the disc 46 near its periphery in a direction parallel to its longitudinal axis L. The valve 40 further includes a multi-lumen resistance catheter 48 that is configured to couple to the drainage or distal catheter at one end and the selection mechanism 44 at the other end. The multi-lumen resistance catheter 48 comprising a set of resistors 48a, each resistor 48a defining a different resistance to flow and being configured as a passage or channel that extends parallel to the longitudinal axis L of the disc 46 and catheter 48.

In general, the resistance of the valve 40 is adjusted by rotating the actuator 42 in order to align the aperture 46a of the disc 46 of the selection mechanism 44 with a resistor 48a. That is, the actuator 42 enables the relative positioning of the selection mechanism 44 with respect to the resistance system 48 by a rotational movement of the disc 46 to axially align the aperture 46a with one of the resistors 48a of the resistance system 48. The disc 46 is positioned so as to allow the flow of CSF to traverse the aperture 46a and resistor 48a and exit through the valve 40. The actuator 42 can comprise a motor connected to the disc 46 in order to drive the rotational movement of the disc 46 with respect to the catheter 48. The size of the aperture 46a should be chosen so as not to limit the resistance of the resistor 48a.

As illustrated in FIGS. 2A and 2B, the selection mechanism 44 and catheter 48 together form an essentially cylindrical shape. Within the catheter 48, the resistors 48a are disposed so as to form a set of passages parallel to each other and to the longitudinal axis of the catheter 48 and the disc 46. The openings of the resistors 48a face the rotational path of the passage 46a of disc 46. In order to provide a set of resistors 48a having different resistances, the resistors 48a are each configured with the same length and a different internal diameter. The resistors 48a can be configured with relatively large diameters to reduce their propensity to clog. Since the resistors 48a are contained within a catheter 48 which provides them with structural integrity, the lengths of each of the resistors 48a can be as long as necessary to achieve the desired resistance. The resistors 48a of the catheter 48 are disposed on a circular trajectory so as to face the rotational path of the passage 46a of the disc 46.

During operation, if the external system controller device 60 detects that the measured value of the physiological characteristic is higher or lower than the preset target value for that characteristic, the device sends a command to the actuator 42 to rotate the disc 46 in order to align the aperture 46a with a selected resistor 48a having a higher or lower resistance than the currently used resistor 48a. For instance, if the measured value is higher than the target value, then the disc 46 is rotated so as to select a resistor 48a having a lower resistance. The selection can be done incrementally, i.e., the disc 46 can be rotated stepwise until the appropriate resistance is attained and the system controller device 60 detects that the measured value is approaching or has approached the target value for that patient. This stepwise adjustment of resistance enables the present invention to operate effectively even when, over time and with use, the resistors 48a become clogged with particulate matter such as blood cells, a potential problem with the resistors 48a having small internal diameters. Since the present invention relies upon the relative resistance or inner diameter of each resistor 48a to operate, rather than the absolute size or resistance level of any particular resistor 48a, the catheter 48 is still effective even when some of the resistors 48a are clogged. This is because the user can bypass (i.e., move incrementally past) any clogged resistors 48a during the adjustment procedure and rotate the catheter 48 until a suitable resistor 48a having either a higher or lower resistance relative to the current resistance is detected. If no difference is detected between the measured value and the target value, then the system controller device 60 is programmed to make no changes and maintain the current resistance of the valve 40.

In general, the internal diameters of the resistors 48a of the multi-lumen resistance catheter 48are chosen so as to provide a range of resistance to CSF flow, preferably between about 0 - 50 mm Hg/ml/min. For example, the internal diameters of the resistors 48a can be in the range of approximately 0.30 mm to about 0.60 mm. The valve 40 is configured such that turning the disc 46 adjusts the resistance to CSF flow through the valve 40. The adjustment process can be done after implantation and non-invasively by means of the actuator 42. As an example, in valve 40 the rotation of the actuator 42 positions the disc 46 to allow the CSF to pass through the desired resistor or passage 48a of the catheter 48 when the resistor 48a is aligned with the aperture 46a of the disc 46. The ability to adjust the resistance to flow of the valve 40 non-invasively after implantation enables the surgeon to optimally fit the patient physiology and provide an optimal treatment of the patient's pathology, in order to match CSF flow rate according to the resorption rate of the drainage site.

Also disclosed but outside the scope of the present invention is a method of regulating the drainage of cerebrospinal fluid from the cranial cavity of the patient in response to changes in the patient's ventricular volume. The method involves adjusting a resistance of an implanted shunt system so that the drainage rate is responsive to ventricular pressure and the volume of the patient's ventricular cavity stays constant. The method may comprise the steps of managing CSF flow in a hydrocephalus patient using the implantable shunt system 30 and external system controller device 60 described herein.

In a hydrocephalus patient 10 having an implanted shunt system 30 of the present invention, either the patient or the attending physician can selectively operate the system controller device 60 by applying the device 60 to the patient 10 to energize the implanted shunt system 30. The system controller device 60 is configured to detect a value of the physiological characteristic of the ventricular cavity, as measured by the sensor element 50. The measured value of the physiological characteristic is compared to a predetermined target value for that physiological characteristic. The predetermined target value can be determined through clinical assessment of the patient and is therefore customized for each particular patient. This target value is then programmed or preset into the system controller device 60. When, or if, the system controller device 60 detects a difference between the measured value and the target value, the device 60 then determines whether the resistance for the valve should be increased or decreased accordingly in order to d achieve the predetermined target value for that physiological characteristic. Using the system controller device 60, the current resistance of the valve 40 is adjusted in order to achieve the desired resistance. If the measured value is essentially the same as, or within an acceptable range of the target value, then no is made change to the resistance of the valve.

During the operation of the external system controller device 60 (i.e., when the device is applied to the patient 10 and the implantable shunt system 30 is energized), data is communicated between the device 60 and the implantable shunt system 30. The sensor element 50 communicates data representative of the measured value of the physiological characteristic to the system controller device 60, and the system controller device 60 communicates a command to adjust the resistance to the valve 40. For example, the system controller device 60 can detect a value of the physiological characteristic measured by the sensor element 50 by receiving an input signal generated from the sensor element 50 that contains data about the measured value of the physiological characteristic. Similarly, the system controller device 60 can adjust the resistance of the valve 40 by generating and transmitting an output control signal to the valve 40 that commands the valve 40 to adjust its resistance.

In one aspect of the disclosed method, the physiological characteristic to be measured is ventricular volume, and the sensor element 50 is a volume sensor configured to measure a volume of the ventricular cavity 12 of the patient. The method is therefore especially useful for managing CSF flow in a patient afflicted with normal pressure hydrocephalus, which is characterized by fluctuations in ventricular volume. It is contemplated that the method can be performed when the patient 10 becomes symptomatic of normal pressure hydrocephalus, i.e., becomes sick, or experiences discomfort or disorientation. The method can also be repeated whenever the patient 10 becomes symptomatic, or ill. Thus, when the patient 10 manifests symptoms of the disease, or becomes ill, the external system controller device 60 can be applied to the patient 10 to detect any variations in the ventricular volume. If the volume has varied from the target volume determined for this patient 10 and which has been preset in the device 60, then the present method can be carried out to adjust the resistance of the valve 40 in an attempt to restore the ventricular volume back to its target volume.

In another aspect, the value of another physiological characteristic of the ventricular cavity 12 can also be detected. For instance, the implantable shunt system 30 can be provided with a second sensor element for measuring an additional physiological characteristic. The second sensor element 52 can be, for example, a pressure sensor, and the second physiological characteristic can be ventricular pressure. Thus, the method can involve the detection of either ventricular volume or ventricular pressure, or both, and assessing the variations in either or both of these physiological characteristics when adjusting the resistance of the valve 40.

In an application of the disclosed method, the patient 10 experiences discomfort and pain. The system controller device 60 is applied onto the patient 10 so that the shunt system 30 is energized and data is communicated from the shunt system 30 to the device 60. The device 60 can be applied by either the patient himself or his attending physician. If the measured value is the same as, or falls within an acceptable range of the target value, then the system controller device 60 is programmed to make no changes to the resistance. If, however, the external system controller device 60 detects that the measured ventricular volume is higher or lower than the preset target ventricular volume, the device 60 sends a command to the actuator 42 to rotate the disc 46 in order to align the aperture 46a with a selected resistor 48a having a higher or lower resistance than the currently used resistor 48a. For instance, if the measured ventricular volume is higher than the target ventricular volume, then the device 60 commands the disc 46 to rotate an increment so as to select a resistor 48a having a lower resistance. Then, after some time has elapsed (e.g., a day, two days, a week, etc.) sufficient to allow the patient's physiology to respond to the valve's new resistance setting, and the patient still experiences discomfort or pain, or simply wants to determine the current ventricular volume, the system controller device 60 can again be applied to the patient 10 to measure a current ventricular volume. If the device 60 does not detect a change in the measured ventricular volume from the previous reading, the device 60 sends another command to the valve 40 to decrease the resistance by rotating the disc 46 another increment.

It is contemplated that the above steps can be repeated until an appropriate resistance is attained and the system controller device 60 detects that the measured ventricular volume is approaching or has approached the target ventricular volume for that patient 10. For example, the above steps can be repeated whenever the patient begins to experience pain or discomfort. However, to safeguard against repeated or excessive valve adjustments within a short window of time, which could produce deleterious health consequences for the patient, the system controller device 60 can include a timed shutoff mechanism which would limit the user's ability to adjust the valve 40 with the system controller device 60 in a given time period. For example, the system controller device's valve adjustment features can be configured to deactivate after each use until a preset amount of time (e.g., a day, two days, a week, etc.) has passed whereby the valve adjustment feature is automatically reactivated. Such a safeguard ensures that a sufficient amount of time passes between adjustments so that the patient's physiology does not incur rapid CSF flow changes in a short amount of time. Of course, it is contemplated that the system controller device 60 can still be capable of detecting the volume of the patient's ventricular cavity even when the device's valve adjustment features are not active. Hence, the patient can continue to monitor his ventricular volume using the system controller device 60 even between stages of adjusting the valve 40.

Thus, there has been described a method of regulating cerebrospinal fluid flow in a hydrocephalus patient, comprising:
providing an implantable shunt system having an adjustable resistance valve for regulating the flow of cerebrospinal fluid into and out of a ventricular cavity of the patient and including a sensor element for measuring a physiological characteristic of the ventricular cavity, and a selectively operable external system controller device for communicating with the implantable shunt system, the system controller device being configured to effect an adjustment of the resistance of the valve when the device is applied to the patient;
energizing the implantable shunt system with the system controller device;
detecting a value of the physiological characteristic of the ventricular cavity measured by the sensor element;
comparing the measured value with a predetermined target value for that physiological characteristic;
determining a desired resistance to achieve the predetermined target value for that physiological characteristic; and
adjusting a current resistance of the valve to achieve the desired resistance.

Preferably, the step of detecting a value of the physiological characteristic comprises communicating data representative of the measured value of the physiological characteristic from the sensor element to the system controller device.

Preferably, the step of communicating includes receiving an input signal generated from the sensor element with the system controller device.

Preferably, the step of adjusting a current resistance comprises communicating a command to adjust the resistance from the system controller device to the valve.

Preferably, the step of adjusting a current resistance is repeated until the predetermined target value is reached.

Preferably, the step of communicating includes transmitting an output control signal generated from the system controller device to the valve.

Preferably, the step of determining a desired resistance includes determining whether an increase or decrease in the current resistance is necessary to achieve the predetermined target value.

Preferably, the step of adjusting a current resistance is repeated after a period of time has elapsed sufficient for the patient to respond to the current resistance of the valve.

Preferably, the physiological characteristic is volume, and the sensor element is configured to measure a volume of the ventricular cavity.

Preferably, the method further includes the step of detecting the value of an additional physiological characteristic of the ventricular cavity.

Preferably, the implantable shunt system includes a second sensor element for measuring the additional physiological characteristic.

Preferably, the second sensor element is a pressure sensor, and the additional physiological characteristic is ventricular pressure.

Preferably, the method is used to manage cerebrospinal fluid flow in a patient afflicted with normal pressure hydrocephalus.

Preferably, the step of energizing the implantable shunt system occurs after the patient becomes symptomatic of normal pressure hydrocephalus.

Preferably, the method is repeated when the patient becomes symptomatic of normal pressure hydrocephalus.

Preferably, the method is repeated after a period of time has elapsed sufficient for the patient to respond to the current resistance of the valve.

While the present invention has been described and illustrated in relation to the treatment of normal pressure hydrocephalus, the method and apparatus described herein are equally suitable for the treatment of other neurological disorders that result in hydrocephalus. It will be understood that the foregoing is only illustrative of the principles of the invention, and that various modifications can be made by those skilled in the art without departing from the scope of the invention.

## Claims

1. An apparatus (20) for regulating cerebrospinal fluid flow in a hydrocephalus patient, comprising:
an implantable shunt system (30) having an adjustable resistance valve (40) for regulating the flow of cerebrospinal fluid into and out of a ventricular cavity (12) of the patient, and including a sensor element (50) for measuring a physiological characteristic of the patient; and
a selectively operable external system controller device (60) for communicating with the implantable shunt system, the system controller device being configured to effect an adjustment of the resistance of the valve when the device is applied to the patient,
**characterised in that** the sensor element (50) is a volume sensor for detecting volumetric variations within the ventricular cavity (12).

2. The apparatus of claim 1, wherein the sensor element (50) is coupled to the valve (40).

3. The apparatus of claim 1 or claim 2, wherein the system controller device (60) is configured to receive an input signal generated from the sensor element (50) during operation, the input signal being representative of a measured volume of the ventricular cavity (12).

4. The apparatus of claim 3, wherein the system controller device (60) is further configured to transmit to the valve (40) an output control signal that commands the valve to adjust the resistance during operation.

5. The apparatus of claim 4, wherein the system controller device (60) includes a microprocessor for comparing the measured volume detected by the volume sensor (50) to a predetermined target volume for the patient.

6. The apparatus of claim 5, wherein the target volume is determined through clinical assessment of the patient and the microprocessor is preprogrammed with the target volume prior to the application of the device to the patient.

7. The apparatus of claim 5, wherein the microprocessor is programmed to calculate a desired resistance for the valve to achieve the target volume.

8. The apparatus of claim 7, wherein the implantable shunt system (30) further includes a second sensor element (52) for measuring an additional physiological characteristic of the patient, the second sensor element being configured to transmit data representing the measured value of the additional physiological characteristic to the system controller device (60).

9. The apparatus of claim 8, wherein the second sensor element (52) is a pressure sensor and the additional physiological characteristic is ventricular pressure.

10. The apparatus of any one of claims 1 to 9, wherein the adjustable resistance valve (40) is configured for implantation in a peritoneal cavity (14) of the patient.

11. The apparatus of claim 4, wherein the system controller device (60) further includes a timed shutoff mechanism.

## Patentansprüche

1. Vorrichtung (20) zum Regeln des Flusses cerebrospinalen Fluides in einem Hydrocephalus-Patienten, die aufweist:
ein implantierbares Ausweich-System (30), das ein Ventil (40) mit einstellbarem Widerstand zum Regulieren des Flusses cerebrospinalen Fluides in einen und aus einem ventrikularen Hohlraum (12) des Patienten hat und ein Sensorelement (50) zum Messen einer physiologischen Eigenschaft des Patienten umfaßt; und
eine wahlweise betreibbare, externe Systemcontrollervorrichtung (60) zum Kommunizieren mit dem impantierbaren Ausweich-System, wobei die Systemcontrollervorrichtung so ausgestaltet ist, daß sie eine Einstellung des Widerstands des Ventils bewirkt, wenn die Vorrichtung bei dem Patienten eingesetzt wird,
**dadurch gekennzeichnet, daß** das Sensorelement (50) ein Volumensensor zum Erfassen volumetrischer Änderungen innerhalb des ventrikularen Hohlraums (12) ist.

2. Vorrichtung nach Anspruch I, bei der das Sensorelement (50) an das Ventil (40) gekoppelt ist.

3. Vorrichtung nach Anspruch 1 oder 2, bei dem die Systemcontrollervorrichtung (60) so geschaltet ist, daß sie ein Eingangssignal empfängt, das während des Betriebes von dem Sensorelement (50) erzeugt wird, wobei das Eingangssignal für ein gemessenes Volumen des ventrikularen Hohlraums (12) repräsentativ ist.

4. Vorrichtung nach Anspruch 3, bei der die Systemcontrollervorrichtung (60) weiter so gestaltet ist, daß sie an das Ventil (40) ein Ausgangssteuersignal sendet, das es dem Ventil befiehlt, während des Betriebes den Widerstand anzupassen.

5. Vorrichtung nach Anspruch 4, bei der die Systemcontrollervomchtung (60) einen Mikroprozessor zum Vergleichen des gemessenen Volumens, das von dem Volumensensor (50) erfaßt worden ist, mit einem vorbestimmten Zielvolumen für den Patienten umfaßt.

6. Vorrichtung nach Anspruch 5, bei der das Zielvolumen durch klinische Bewertung des Patienten bestimmt wird und der Mikroprozessor vor dem Einsatz der Vorrichtung bei dem Patienten mit dem Zielvolumen vorprogrammiert wird.

7. Vorrichtung nach Anspruch 5, bei der der Mikroprozessor so programmiert ist, daß er einen gewünschten Widerstand für das Ventil berechnet, um das Zielvolumen zu erreichen.

8. Vorrichtung nach Anspruch 7, bei der das implantierbare Ausweich-System (30) weiter ein zweites Sensorelement (52) zum Messen einer zusätzlichen physiologischen Eigenschaft des Patienten umfaßt, wobei das zweite Sensorelement so ausgestaltet ist, daß es Daten, die den gemessenen Wert der zusätzlichen physiologischen Eigenschaft darstellen, an die Systemcontrollervorrichtung (60) sendet.

9. Vorrichtung nach Anspruch 8, bei der das zweite Sensorelement (52) ein Drucksensor ist und die zusätzliche physiologische Eigenschaft ein ventrikularer Druck ist.

10. Vorrichtung nach einem der Ansprüche 1 bis 9, bei der das Ventil (40) mit einstellbarem Widerstand zum Implantieren in einen peritonealen Hohlraum (14) des Patienten ausgestaltet ist.

11. Vorrichtung nach Anspruch 4, bei der die Systemcontrollervorrichtung (60) weiter einen zeitlich gesteuerten Abschaltmechanismus umfaßt.

## Revendications

1. Dispositif (20) destiné à réguler l'écoulement du fluide céphalorachidien d'un patient présentant une hydrocéphalie, comprenant :
◆ un système de dérivation implantable (30) ayant une soupape à résistance réglable (40) destinée à réguler l'écoulement de fluide céphalorachidien dans et hors d'une cavité ventriculaire (12) du patient et comprenant un élément capteur (50) destiné à mesurer une caractéristique physiologique du patient ; et
◆ un dispositif de commande de système externe sélectivement actionnable (60) destiné à communiquer avec le système de dérivation implantable, le dispositif de commande de système étant configuré pour effectuer un réglage de la résistance de la soupape lorsque le dispositif est appliqué au patient,
**caractérisé en ce que** l'élément capteur (50) est un capteur de volume destiné à détecter des variations volumétriques dans la cavité ventriculaire (12).

2. Dispositif selon la revendication 1, dans lequel l'élément capteur (50) est couplé à la soupape (40).

3. Dispositif selon la revendication 1 ou la revendication 2, dans lequel le dispositif de commande de système (60) est configuré pour recevoir un signal d'entrée généré depuis l'élément capteur (50) en fonctionnement, le signal d'entrée étant représentatif du volume mesuré de la cavité ventriculaire (12).

4. Dispositif selon la revendication 3, dans lequel le dispositif de commande de système (60) est en outre configuré pour transmettre à la soupape (40) un signal de commande de sortie commandant la soupape pour régler la résistance en fonctionnement.

5. Dispositif selon la revendication 4, dans lequel le dispositif de commande de système (60) comprend un microprocesseur destiné à comparer le volume mesuré détecté par le capteur de volume (50) à un volume cible prédéterminé pour le patient.

6. Dispositif selon la revendication 5, dans lequel le volume cible est déterminé par évaluation clinique du patient et le microprocesseur est préprogrammé avec le volume cible avant l'application du dispositif sur le patient.

7. Dispositif selon la revendication 5, dans lequel le microprocesseur est programmé pour calculer une résistance souhaitée de la soupape pour obtenir le volume cible.

8. Dispositif selon la revendication 7, dans lequel le système de dérivation implantable (30) comprend en outre un second élément capteur (52) destiné à mesurer une caractéristique physiologique supplémentaire du patient, le second élément capteur étant configuré pour transmettre des données représentant la valeur mesurée de la caractéristique physiologique supplémentaire au dispositif de commande de système (60).

9. Dispositif selon la revendication 8, dans lequel le second élément capteur (52) est un capteur de pression et la caractéristique physiologique supplémentaire est la pression ventriculaire.

10. Dispositif selon l'une quelconque des revendications 1 à 9, dans lequel la soupape à résistance réglable (40) est configurée pour implantation dans une cavité péritonéale (14) du patient.

11. Dispositif selon la revendication 4, dans lequel le dispositif de commande de système (60) comprend en outre un mécanisme d'arrêt temporisé.
